# EUROPEAN PATENT APPLICATION

(11) **EP 0 771 867 A2**
(43) Date of publication of application: **07.05.1997**
(21) Application number: 96810200.4
(22) Date of filing: 01.04.1996
(51) Int. Cl.: C12M 1/40, C12Q 1/26, C12Q 1/00

(54) **Enzyme electrode**

(30) Priority: 30.10.1995 DE 95810670 U; 30.11.1995 DE 95810752 U
(71) Applicant: Japat Ltd, CH-4057 Basel (CH)
(72) Inventor: Asakura, Toshikage, Osaka (JP); Yamato, Hitoshi, Hyogo (JP); Ohwa, Masaki, Kobe City 657 (JP); Khan, Golan Faruque, Kobe-shi, Hyogo (JP)
(74) Representative: Dannappel, Hans-Jochen

(57) **Abstract**

Disclosed is an enzyme electrode which comprises
(a) an electroconductive support member (ESM) comprising a porous electroconductive layer,
(b) an enzyme adsorbed or immobilized onto the surface of said porous layer in an catalytically effective amount, and
(c) a protecting layer to prevent leaching of said enzyme from the porous layer.

The electrode may be used for indicating amperometically the catalytic activity of an enzyme in the presence of a liquid containing substance acted upon by the enzyme and to be detected, and in the presence of an electric potential on the electrode.

## Description

This invention relates to an enzyme electrode which can be used as an amperometric biosensor, a chemical sensor or in a bioreactor, and to a process for amperometrically detecting chemicals. More specifically, it relates to enzyme electrodes having extended working and shelf lives.

As an enzyme electrode used as a biosensor which responds amperometrically to the catalytic activity of the enzyme in the presence of a substrate, several types have been known as described in Biotech. Genet. Eng. Rev., 1, pp. 89-120 (1984), et. al. and specific examples thereof have been proposed, for example, in U.S. Patents No. 4,970,145 and No. 5,160,418.

In U.S. Patent No. 4,970,145, there has been disclosed enzyme electrodes for indicating amperometrically the catalytic activity of an enzyme in the presence of a liquid containing a substrate acted upon by the enzyme and of an electric potential on the electrode which comprise (a) an electroconductive support member (hereinafter referred to as "ESM") comprising a porous electroconductive layer formed of carbon particles, a platinum group metal bonded by resin, and (b) a catalytically active quantity of the enzyme adsorbed or immobilized onto the surface of the porous substrate. Also, in U.S. Patent No. 5,160,418, there has been disclosed a method for the manufacture of enzyme electrode comprising a support and an electroconductive layer formed thereon containing a finely divided platinum group metal or oxide and an enzyme deposited on the surface of the underlying support.

In the above prior art, enzyme electrodes using very small quantities of immobilized enzyme and showing much-improved response and stability have been obtained. However, in the above techniques of the prior art, stability of the electrodes has not necessarily been high since the enzyme and the electroactive metal particles exist in the same layer whereby the catalytic activity of the enzyme is likely lost when manufacturing the electrode, or adsorbed or immobilized enzyme is lost with a lapse of time during measurement.

An object of the present invention is to provide an enzyme electrode having a high sensitivity, an extended linearity and a low interference current as a biosensor, and easily prepared with a low cost, particularly having an extremely extended working life, shelf life and storage stability.

According to the present invention, there is provided an enzyme electrode which comprises
(a) an electroconductive support member (ESM) comprising a porous electroconductive layer,
(b) an enzyme adsorbed or immobilized onto the surface of said porous layer in an catalytically effective amount, and
(c) a protecting layer to prevent leaching of said enzyme from the porous layer.

Adsorbed or immobilized means also deposited onto the surface of the particles forming the porous layer.

The ESM may be composed of an electrical conductive support on a surface of which the porous layer is deposited or coated. Conductive supports may be metals, composites of a dielectric support and a metal layer or a conductive metal oxide layer like indium oxides (ITO glass).

The enzyme electrode according to the present invention can be used for amperometric biosensors, biosensors, chemical sensors or in bioreactors.

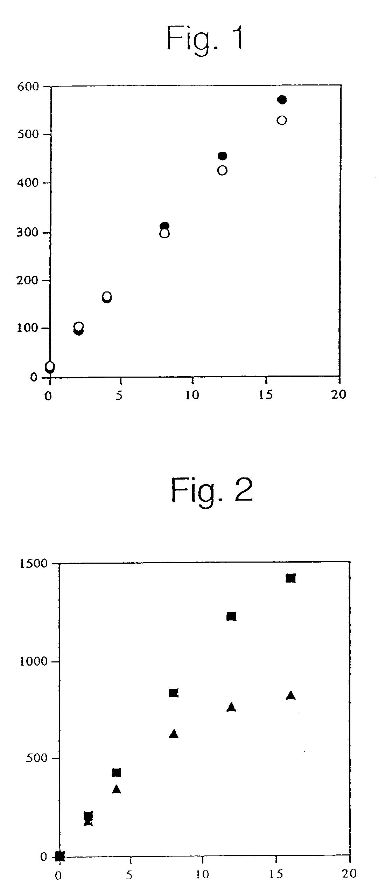

Figure 1 illustrates a calibration curve against lactate concentration wherein assay is carried out by stopped-flow analysis at 37 °C and lactate sensors are polarized at 400 mV vs. Ag/Agcl (64 µg/cm² of the enzyme is loaded). The x-axis shows the lacteate concentration in mM and the y-axis shows the current in nA. The hollow circle is the 3 day and the filled circles is 59 day measurement.

Figure 2 illustrates a calibration curve against lactate concentration wherein assay is carried out by stopped-flow analysis at 37 °C and lactate sensors are polarized at 400 mV vs. Ag/Agcl. The x-axis shows the lacteate concentration in mM and the y-axis shows the current in nA. The squares means the 1 day and the triangles means the 10 day measurement.

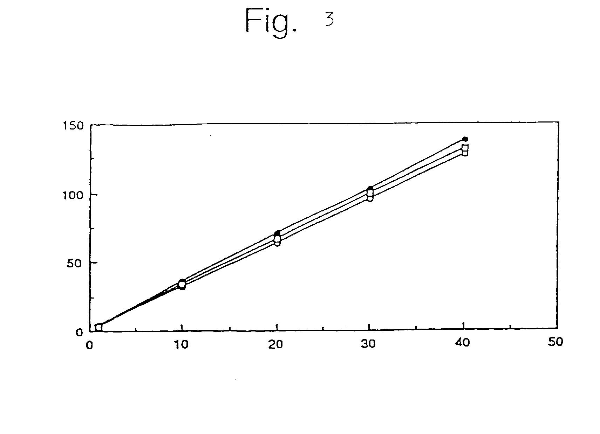

Figure 3 illustrates a calibration curve against glucose concentration wherein glucose sensors are polarized. The x-axis shows the glucose concentration in mM and the y-axis shows the response current in µM. Hollow circles are the 1 day, filled circles the 22 day and the hollow squares the 42 day measurment.

In the following, the present invention will be explained in more detail.

In the present invention, it is also provided a process for indicating amperometrically the catalytic activity of an enzyme contained in the active coating of an enzyme electrode in the presence of a liquid containing a substance acted upon by said enzyme and of an electric potential on the electrode, the improvement which comprises an electrode having (a) an electroconductive support member comprising a porous electroconductive layer, (b) said enzyme adsorbed or immobilized onto the surface of said porous layer in an catalytically effective amount, and preferably 10 to 3000 µg/cm², and (c) a stabilizing layer to prevent leaching out of said enzyme from the porous layer.

The method may be used for the quantitative or qualitative analyses of organic or bioorganic compounds which respond to the catalytical activity of an enzyme used in the porous layer.

The ESM may comprise carbon particles, a platinum group metal and a binder resin. In intimate surface of the carbon particles, finely divided particles of a platinum group metal are contacted therewith and the carbon particles are bonded by the resin. The ESM constitutes a substantially heterogeneous porous substrate consisting essentially of resin-bonded metalised carbon particles with said metal particles distributed substantially uniformly therethroughout in a substantially colloidal state.

The carbon particles to be used in the present invention may be any material so long as they easily permit the subsequent adsorption or immobilization of the enzyme and are electroconductive. The carbon particles to be used in the present invention may be obtained by the furnace process or the contact process, preferably by the furnace process. Specifically, they may include, for example, commercially available carbon black, graphite powder, acetylene black, etc. such as Vulcan XC-72 (trade name, available from Cabot Co., Ltd.; oil absorption: 185 cc/100 g), Ketjen black EC (trade name, available from Lion Akzo Co., Ltd.; oil absorption: 350 cc/100 g), Conductex SC (trade name, available from Columbia Carbon Co., Ltd.; oil absorption; 115 cc/100 g) and the like. These carbon particles preferably have a particle size of about 50 to about 300 Angstroms (about 5 to 30 nm), an oil absorption measured by the DBP process of 100 cc per 100 g or more and a specific surface area measured by the BET method of 10 to 200 m²/g.

The platinum group metal to be used in the present invention is preferably present in the form of particles adsorbed onto the surface of individual carbon particles and may include platinum, palladium, ruthenium, rhodium, osmium and iridium, particularly preferably platinum or palladium. The platinum group metal is preferably present in the form of particles more finely divided in particle size than those of the carbon particles, and present in the form of particles adsorbed onto the surface of individual carbon particles, and metal particles preferably have colloidal sizes in the range of about 10 to 100, more preferably 10 to 50 and in particular 15 to 25 Angstroms (1 to 10, 1 to 5 or 1.5 to 2.5 nm). The platinum group metal is preferably present in an amount of 1 to 100 % by weight, more preferably 5 to 80 % based on the weight of the carbon particles.

For preparing the platinum group metal particles with a more finely divided or colloidal particle size, there may be employed a method mentioned below as described in U.S. Patent No. 4,044,193. That is, chloroplatinic acid is neutralized with sodium carbonate to form orange-red Na₂Pt(Cl)6, then sodium bisulfite is added thereto to drop the pH to about 4, and more sodium carbonate is added to bring the pH back to neutral. It is slurried with water, and then enough strong acid resin added, the solution is filtered to remove the resin and passed through an ion-exchange column with sufficient of the said acid resin to replace the other three Na-atoms. The solution is concentrated by boiling to obtain a complex sulfite platinum acid compound. This complex is decomposed by heating to dryness in air (oxidization) and holding the temperature at about 135 °C for about one hour to give a colloidal platinum-containing sol having an average finely divided platinum particle size. The sol can be then deposited or adsorbed on carbon particles according to the conventional manner. In the present invention, commercially available platinised activated carbon, such as supplied from E-TEK Inc., MA, USA, may be used.

The resin to be used as a binder according to the present invention is not specifically limited so long as it has sufficient characteristics to be required for the support member of the enzyme electrode but may preferably include a polytetrafluoroethylene (PTFE), a synthetic fluorocarbon resin other than PTFE, an (meth)acrylic resin (e.g., poly(ethyl methacrylate), poly(methyl methacrylate) and other copolymers of (meth)acrylic acid and/or (meth)acrylate), a polystyrene resin, a poly(vinyl acetate), a poly(vinyl chloride), a polycarbonate, poly(4-methyl-1-pentene), polyisoprene, polychloroprene, poly(1,3-butadiene), a silicone rubber, and a poly(ethylene terephthalate). Among these, particularly preferred are polyesters, such as poly(ethylene terephthalate), or poly(vinyl chloride).

In the present invention, the ESM may comprise (a') an electroconductive polymer film and (a") a layer comprising particles of a platinum group metal as mentioned above. The electroconductive polymer film may be composed of a polyheteroaromatic polycation or a polyanaline polycation and non nucleophilic small anions like halogenides, BF₄, PF₆, AsF₆, SbF₆, or polymeric polyanions of polymers with -SO₃ or -OSO₃⁻groups. Examples for polycations are unsubstituted or C₁-C₄alkyl C₁-C₄al-koxy or C₂C₆alkylenedioxyl substituted polypyrroles, polythiophenes or polyanilines. These electroconductive materials are well known in the prior art. Among them, a polypyrrole film is particularly preferred. Most preferred, the polypyrrole film is shapable. Examples of such a shapable polypyrrole films with polysulfated polymeric anions (hereinafter referred to as "SEC film") are described in, for example, U.S. Patent Re. 34,514. For the platinium metal particles the same preferred embodiments are applied as above and in the follwing.

It was surprisingly found that the particles of a platinum group metall well adhere to the electroconductive polymer even without a binder. Further surprisingly the metal colloid must not be in contact with carbon particles. A further subject of the invention is an electroconductive polymer, comprising onto at least one surface a layer of finely divided particles of a platinum group metal. The layer may have the same porosity as in the above described ESM, with or without binder.

When the ESM comprises an electroconductive polymer film and a layer comprising platinum group metal particles, the layer comprising platinum group metal particles may be formed by electrochemical metallisation, heat-pressing or casting with a polymer binder, which are conventionally known in the art.

The electrochemical metallisation can be achieved, for example, by cathodic deposition of Pt black on an electroconductive polymer film in an aqueous solution of potassium hexachloro-platinate. The heat-pressing can be carried out, for example, by casting Pt black particles on an electroconductive polymer film from a suspension in an alkoxyalcohol such as 2-methoxyethanol, followed by drying then pressing the film, such as by two heated rollers (temperature: 150 to 160 °C). The casting can be accomplished, for example, by casting a Pt black paste made with a polymer on an electroconductive polymer film, and then drying.

In a preferred embodiment of the invention the ESM comprises platinised activated carbon treated with bovine serum albumin (BSA) and a binder resin which layer preferably has a thickness of about 1 to 50 µm, more preferably 3 to 40 µm. The BSA treatment described here is preferred to make the ESM hydrophilic.

In a further preferred embodiment of the invention the porous ESM is preferred, particularly the macroporous ESM is preferred. The macroporous ESM comprises pores which have about 1 to 50 µm average diameter.

The ESM detects H₂O₂ generated by the enzyme deposited, adsorbed or immobilized onto the surface of the porous layer.

The enzyme to be used in the present invention may be deposited, adsorbed or immobilized onto the surface of said porous layer in an amount of 10 to 3000 µg/cm², preferably in an amount of 30 to 1500 µg/cm². If the amount is less than 10 µg/cm², good linearity of a calibration curve for the analyte cannot be obtained, while if it exceeds 3000 µg/cm², no additional effect can be obtained. The enzyme is deposited, adsorbed or immobilized by casting or physical adsorption. As a method for deposition, adsorbance or immobilisation the enzyme onto the surface of the porous layer, there may be mentioned an adsorption method in which an enzyme is dissolved in a buffer and the solution is dispensed on the support or an immobilisation method such as glutaraldehyde treatment, carbodiimide treatment, carbonyldiimidazole treatment or DFDNB (3,4-difluoro-1,6-dinitrobenzene) treatment as described in U.S. Patent No. 4,970,145.

The enzyme to be used in the present invention may include, for example, an oxidoreductase such as glucose oxidase, lactate oxidase, cholesterol oxidase, choline oxidase, glutamate oxidase, pyruvate oxidase, lactose oxidase, oxalate oxidase and sarcosine oxidase, preferably lactate oxidase and sarcosine oxidase. In addition, the oxidoreductase may be accompanied by at least one enzyme other than the oxidoreductase. Such an enzyme other than the oxidoreductase may include creatininase with creatinase. In the present invention, the enzyme is particularly preferably sarcosine oxidase and the enzyme other than oxidoreductase is creatininase with creatinase.

The protection layer is formed on the ESM and the enzyme to prevent leaching of the enzyme therefrom. This layer is preferably hydrophilic. By providing the stabilizing layer, leaching of the enzyme can be remarkably prevented whereby stability of an electrode can be markedly improved. As a material for forming the protection layer, there may be mentioned, for example, at least one selected from the group consisting of gelatin, bovine serum albumin, poly(vinyl alcohol), poly(ethylene oxide), poly(vinyl pyrrolidone), polyacrylamide and poly(2-hydroxyethyl methacrylate) each of which may have a substituent such as a stylbazolium group, vinyl group, etc. Among them, preferred are gelatin, poly(vinyl alcohol), poly(ethylene oxide) and poly(vinyl pyrrolidone). The protection layer function is to stabilize the enzym containing ESM.

The stabilizing layer may be crosslinked after providing the layer on the enzyme by using glutaraldehyde or a photo cross-linker. As the photo cross-linker, there may be used any materials so long as they generate a radical by irradiation of light and may be mentioned, for example, Irgacure 2959, Irgacure 907 and Irgacure 369 (all tradenames, available from Ciba Geigy AG). The stabilizing layer preferably has a thickness of 1 to 100 µm, more preferably 3 to 50 µm.

In the present invention, a protective cover layer for covering the protection layer may be provided on the stabilizing layer to regulate the amount of the analyte to be permeated therethrough. The protective cover layer may comprise any material which can protect the inner layers and regulate the amount of the analyte to a suitable amount. As the material for forming the protective cover layer, there may be mentioned, for example, at least one selected from the group consisting of a polycarbonate, a polyester, a polyamide, a perfluorinated ion-exchange resin, and the like. Such a protective cover layer can be formed by adhering a membrane or a film onto the stabilizing layer or coating a resin solution onto the same and drying. The protective cover layer preferably has a thickness of 0.05 to 20 µm, more preferably 0.1 to 10 µm, and has an average pore size of 1 to 50 nm, more preferably 5 to 30 nm.

The above enzyme electrode can be used as biosensors for detecting an amount of glucose, lactate, creatinine, cholesterol, choline, glutamate, pyruvate, lactose, sucrose, oxalate, acetylcholine, total cholesterol, etc., particularly as an amperometric biosensor, a chemical sensor or in a bioreactor

The biosensor of the present invention preferably comprises (a) an ESM, (b) an enzyme layer, (c) a stabilizing layer and, (d) a protective cover layer.

Such a biosensor can be prepared, for example, as follows. First, a base electrode is prepared. Secondly, colloidal platinum is deposited onto the surface of carbon powder by oxidative decomposition using H₂O₂, and then the platinised activated carbon is treated in phosphate buffer to effect neutralization. The resulting material is then mixed with a binder resin and the mixture is milled and after controlling the viscosity thereof, applied onto the electrode, base electrode, by a screen-printing to form an ESM. An enzyme dissolved in a buffer solution is then dispensed onto the ESM and dried. Then, a solution of a material for forming the stabilizing layer is sprayed over the enzyme layer, and after drying, the resulting material is dipped in, for example, a glutaraldehyde solution to cross-link the stabilizing layer. Further, a film for the protective cover layer is adhered or a resin solution for forming the protective cover layer is coated and dried whereby a biosensor can be prepared.

Biosensors prepared by the above mentioned layer-layer method show an excellent improvement of the performances compared to the state of the art. The developed system works with the most of the H₂O₂ generating enzymes, thus, enables to fabricate a series of biosensors for glucose, lactate, creatinine, cholesterol, choline, glutamate, pyruvate, lactose, sucrose, oxalate, acetylcholine, total cholesterol, etc. by employing the corresponding enzyme/enzymes in the enzyme layer. The performances of the layer-layer structured biosensors are as follows:
1) highly sensitive to the analyte (maximum current: 200 to 1000 µA/cm²), thus, it enables to measure a sub-micromolar concentration of analytes,
2) extended linearity,
3) low interference current, and
4) simple preparation with a low cost.

The most significant improvement of the invention is an extended or elongated working life and shelf life as well as a high storage stability..

The thus prepared biosensors of the present invention show a surprisingly good linearity with an expanded concentration range for a long period of time.

In the following, the present invention will be explained in more detail by referring to examples.

### Example 1: Preparation of porous ESM

About 3.15 g of platinised activated carbon (PAC, 5% platinum), available from E-TEK (Natic, MA, USA) prepared by the deposition of colloidal platinum (particle size: between about 1.5 to 2.5 nm) onto the surface of the carbon powder (nominal particle size: about 30 nm) by oxidative decomposition of complex platinum sulfite acid (II) using H₂O₂, are treated in phosphate buffer to neutralize any residual sulfuric acid present. The phosphate buffer also includes a microbicide, sold under the trademark Kathon CG microbicide (a trademark of Rohm & Haas Corp., Philadelphia, PA). The buffer is prepared by adding 11.499 g of sodium phosphate, dibasic (Na₂HPO₄), 2.898 g of sodium phosphate, monobasic monohydrate (NaH₂PO₄^{.}H₂O) and 1.0 g of Kathon CG microbicide to 1000 ml of distilled water. The buffer formulation is tested using a pH meter and electrode, to have a pH of 7.5. Approximately 100 ml of the phosphate buffer is added to the 3.15 g of PAC, and is mixed for 7 days. The buffer is replaced after the first 3 days of mixing by allowing the PAC to settle, decanting off 60 ml of the used buffer and replacing it with 100 ml of fresh buffer, and is mixed for 7 days. The mixture is then vacuum filtered after the 7 days of mixing, and the neutralized carbon is washed while under vacuum filtration using 100 ml of buffer. The vacuum is maintained for about 15 to 20 seconds after the bulk of the buffer is pulled trough the carbon to slightly dry the carbon and improve handling of the material.

The PAC is then mixed with 625 mg of bovine serum albumin (BSA), available from Sigma (St. Louis, MO, USA). The 625 mg of BSA is first added to the flask containing the PAC and an additional 40 ml of buffer. The BSA and PAC is gently mixed and allowed to sit for 0.5 hr to permit the BSA to dissolve. The mixture is then gently mixed overnight at room temperature. The BSA-PAC mixture is then vacuum filtered and applied for about 20 sec. after the bulk of the buffer is pulled through the BSA-PAC to dry the BSA-PAC to between about 60 to 70 % water content.

The ink is formulated by adding 5.0 g of a binder resin, available as product number 8101 RS (a polyester resin) available from Metech (Elverson, PA, USA) to 2.0 g of the BSA-PAC (as prepared above). To this mixture, 0.25 g of Triton X-100 surfactant, available from Tokyo Kasei (Tokyo, Japan), is added as a printing flow aid. The mixture is then milled using a standard paint industry three roll mill. 1.0 ml of Albesso thinner, available from Metech as 8101 RS thinner, is added to the mixture, after the first milling is completed to adjust the viscosity of the paste for printing purposes. The mixture is then milled for a second period. The resulting paste is then applied for a screen-printing onto the electrode to form working electrode.

### Comparative example 1 : Preparation of Non-porous ESM

The PAC without BSA treatment is prepared according to the procedure described in Example 1 and used to make a non-porous electrode. The ink is formulated by adding 0.250 g of Metech 8101 RS to 0.238 g of the PAC and 0.016 g of Triton X-100. The resulting mixture is then milled as Example 1 and then applied for a screen-printing onto the electrode to form working electrode.

### Example 2: Biosensor Preparation

Lactate oxidase *(Aerococcus Viridans),* 1.0 mg (20% protein content), is dissolved in 100 µL of phosphate buffer solution (0.1 M KH₂PO₄/K₂HPO₄, pH 7.0) to give 2 mg/ml of LOD solution. The resulting solution of 1.0 µL is dispensed on the above ESM (detecting area: 0.0314 cm²) prepared in Example 1 except for applying 40% platinum loaded PAC in place of 5% platinum loaded PAC, which is equivalent to approximately 64 µg/cm² of active enzyme on the electrode surface. After drying for 30 min at room temperature, the electrode is further dried in the oven at 30°C for 1 hr under vacuum. Gelatin solution (5% in deionized water), 10 µl is sprayed over the enzyme layer to form the stabilizing layer. The resulting electrode is dried for 30 min at room temperature and then dried at 30°C under vacuum. The gelatin layer is cross-linked by dipping into 5% glutaraldehyde solution for 30 second. After washing three times with deionized water successively, the electrode is dried at room temperature for 1 hr and then at 30°C under vacuum overnight.

After applying Nuclepore® (pore size: 15 nm, thickness: 6 µm) as a protective cover layer, the performance of the resulting lactate sensor is tested to various lactate concentrations by stopped-flow analysis at 37 °C by polarizing at 400 mV vs Ag/AgCI. 64 µg/cm² of the enzyme was loaded. The results are summarized in Fig. 1. As shown in Fig 1, the response of the electrode is unchanged even after 59 days' continuous operation and showed extremely good linearity with a wide concentration range.

### Comparative example 2:

A lactate sensor based on the prior art is prepared according to the procedure described in Example 1 except for adding lactate oxidase (from *Aerococcus Viridans)* ) into the PAC paste. The resulting paste is applied to a screen printing onto the electrode to form working electrode. After applying Nuclepore® (pore size; 15 nm, thickness: 6 µm) as a protective cover layer, the performance of the resulting lactate sensor is tested to various lactate concentrations at 400 mV vs Ag/Agcl. The results of Day 1 and Day 10 are summarized in Fig. 2. As can be seen from Fig. 2, it can be understood that when an enzyme is used by mixing with a paste, stability with a lapse of time as a biosensor becomes worse and 10 days later, no good linearity can be obtained.

### Example 3:

The procedure of Example 2 is repeated except for applying 0.5 µl of lactate oxidase solution (2 mg/ml) on the electrode surface, which is equivalent to approximately 32 µg/cm² of active enzyme on the electrode surface. Assay is carried out by stopped-flow analysis at 37 °C. Lactate sensors are polarized at 400 mV vs Ag/Agcl. 32 µg/cm² of enzyme was loaded. An essentially similar linear electrode response having extremely good linearity between 0 and 16 mM is obtained with the sensitivity of 2.74 µA/cm² × mM.

### Example 4:

The procedure of Example 2 is repeated except for applying 8 µl of lactate oxidase solution (5 mg/ml) on the electrode surface, which is equivalent to approximately 1280 µg/cm² of active enzyme on the electrode surface. Assay is carried out by stopped-flow analysis at 37 °C . Lactate sensors are polarized at 400 mV vs Ag/Agcl, 1280 µg/cm² of enzyme is loaded. The current output from the electrode material measured at various lactate concentration is observed. An essentially similar linear electrode response having extremely good linearity between 0 and 16 mM was obtained with the sensitivity of 2.80 µA/cm² × mM.

### Example 5:

The procedure of Example 2 is repeated except for using Ho-326 (tradename, available from Toyo Gosei, poly(vinyl alcohol) having stylbazolium group) in place of gelatin-glutaraldehyde. Assay is carried out by stopped-flow analysis at 37 °C . Lactate sensors are polarized at 400 mV vs Ag/Agcl. Enzyme is entrapped by the poly(vinyl alcohol) type prepolymer. The current output from the electrode material measured at various lactate concentration is observed. An essentially similar linear electrode response having extremely good linearity between 0 and 16 mM is obtained with the sensitivity of 2.99 µA/cm² × mM.

### Example 6:

The procedure of Example 2 is repeated except for using ENT-1000 (tradename, available from Kansai Paint, poly(ethylene oxide) having vinyl groups at the both ends) and Irugacure 2959 (available from Ciba-Geigy A.G.) as a photoinitiator in place of gelatin-glutaraldehyde. Assay is carried out by stopped-flow analysis at 37 °C . Lactate sensors are polarized at 400 mV vs Ag/AgCI. Enzyme is entrapped by the poly(ethylene oxide) type prepolymer. The current output from the electrode material measured at various lactate concentration is observed. An essentially similar linear electrode response having linearity between 0 and 16 nM was obtained with the sensitivity of 0.217 µA/cm² × mM.

### Comparative example 3:

Example 2 is repeated except for using the non-porous electrode prepared in Comparative example 2. Assay is carried out by stopped-flow analysis at 37 °C . Lactate sensors are polarized at 400 mV vs Ag/Agcl. Enzyme is loaded on the flat type chips. The current output from the electrode material measured at various lactate concentration under the same conditions is observed. An essentially similar linear electrode response having extremely good linearity between 0 and 10 mM was obtained with the sensitivity of 2.53 µA/cm² × mM. At the lactate concentration over 10 mM no good linearity can be obtained.

### Example 7:

The procedure of Example 2 is repeated except for applying 80% platinum loaded PAC in place of 40% platinum loaded PAC. Assay is carried out by stopped-flow analysis at 37 °C . Lactate sensors are polarized at 400 mV vs Ag/Agcl. Enzyme is loaded on the chips comprising 80 % PAC. The current output from the electrode material measured at various lactate concentration is observed. An essentially similar linear electrode response having extremely good linearity between 0 and 16 mM is obtained with the sensitivity of 0.2 µA/cm² × mM.

### Example 8:

Creatininase (from *Pseudomonas sp.,* Asahi Chemical, 8.8 units/mg), 0.9 mg, creatinase (from *Bacillus sp.,* Asahi Chemical, 7.0 unit/mg), 3.4 mg and sarcosine oxidase (from *Arthrobacter sp.,* Toyobo, 5.7 unit/mg), 1.4 mg are dissolved in 100µl of phosphate buffer solution (0.1 M KH₂PO₄/K₂HPO₄, pH 7.0). 2.0 µl of the resulting solution is dispensed on the above H₂O₂ detecting layer (detecting area : 0.0314 cm²) prepared in Example 1. After drying for 30 min at room temperature, the electrode is further dried in the oven at 30°C for 1 hr under vacuum. Gelatin solution (5% in deionized water), 10 µl is sprayed over the enzyme layer to form the stabilizing layer. The resulting electrode is dried for 30 min at room temperature and then dried at 30°C under vacuum. The gelatin layer is crosslinked by dipping into 5% glutaraldehyde for 30 second. After washing three times with deionized water successively, the electrode is dried at room temperature for 1 hr and then at 30°C under vacuum overnight.

The performance of the resulting creatinine sensor is carried out at various creatinine concentrations. Assay is carried out by the manually injected stopped-flow analysis at room temperature. Creatinine sensors are polarized at 400 mV vs Ag/Agcl. A good linearity is obtained over the creatinine concentration of from 0 to 250 µM with the sensitivity of 13.9 nA/cm² × µM.

### Example 9:

Example 6 is repeated except for using 0.11 mg of creatininase (from *Pseudomonas sp.,* Toyobo, 258 units/mg), 4.8 mg of creatinase (from *Actinobacillus,* Sigma 30 units/mg) and 0.79 mg of sarcosine oxidase (from *Bacillus sp.,* Asahi Chemicals, 36 units/mg). Assay is carried out by the manually injected stopped-flow analysis at room temperature. Creatinine sensors are polarized at 400 mV vs Ag/Agcl. The current output from this electrode is measured under the same conditions of Example 6. A good linearity is obtained over the creatinine concentration from 0 to 100 µM with the sensitivity of 32.3 nA/cm² × µM.

### Example 10: Preparation of platinised shapable electroconductive (P-SEC) film

### (a) Cleaning of SEC film

As the surface of SEC film is highly hydrophobic, it is probable that the film's surface remains dirty due to the adsorption of organic particles. Therefore, to clean the surface, the film is treated overnight in ethanol and washed with acetonitrile for 1 to 2 hours, and finally, washed thoroughly with milli-Q water and vacuum dried for 2 to 3 hours, then, stored at room temperature in a closed container.

### (b) Platinisation

Two different methods are used to prepare a Pt black layer on SEC film.
*1) Electrochemical platinisation:* Platinisation is carried out either potentiostatically or galvanostatically in a solution containing 10 to 50 mM of hexachloroplatinic acid and 0.6 mg/ml of lead acetate. The solution also contains 0.1 ml HCI or 0.1 M H₂SO₄. Potentiostatic platinisation is performed by applying a potential in the range of -0.2 V to -0.4 V (all the potential in this report is against Ag/AgCI) to the SEC film working electrode for 5 to 10 min. On the other hand, galvanostatic deposition is carried out at a fixed current in the range between 2 mA/cm² to 6 mA/cm² for 5 to 10 min. To prepare larger P-SEC film, a horizontal rotating type electrode is used. SEC film is fixed on a rotary cylinder with the help of adhesive tape. Usually, galvanostatic deposition from diluted solution (10 mM H₂PtCl₆) is performed on this SEC film while the electrode is rotated at a speed of 10 to 100 rounds per min. After platinisation the P-SEC is washed overnight with a phosphate buffer solution (PBS) of pH 7 and dried.
*2) Heat-pressed method:* Very fine Pt black powder is prepared by chemical reduction by NaBH₄. A suspension of this powder is made in 2-methoxyethanol. The diluted suspension is spread on the SEC film and dried. This Pt black casted film is pressed by two hot rollers. Temperature of the roller is set at 150 to 160 °C . At this temperature SEC becomes very soft, so Pt black particles strongly adhere with the SEC film and makes a very uniform platinised SEC film.

### Example 11: Biosensor preparation

P-SEC film is cut into pieces of known dimension. 10 to 20 mg/ml of enzyme is dissolved in PBS of pH 7.0. 20 µl/cm² of the GOD solution is spread on the P-SEC film and kept in an ambient condition until the film was dried up. Or, P-SEC is incubated overnight in the above enzyme solution for the adsorption of the enzyme. After solution is prepared in water and incubated 30 min at 37 °C . 10 µl/cm² of gelatin solution is spread over the enzyme casted P-SEC film and dried under ambient condition. This film is then dipped into glutaraldehyde solution (5 % in water) for 60 sec and washed with water thoroughly, and dried for several hours in ambient condition. Thus film is stored at -18 °C until use. The results are summarized in Figure 3 and table 1.

Fig. 3 shows calibration curves of the glucose sensor at the 1st day, 22nd day and 42nd day. Thus, it can be understood from Fig. 3 that the biosensor of the present invention shows good linearity even after 22nd and 42nd days. Also, table 1 shows a storage stability of the layer/layer structured glucose sensor on the P-SEC film. As shown in table 1, slope of the calibration curves of the glucose sensor according to the present invention is extremely stable even after 15 months storage.

**Table 1**

| STORAGE (MONTHS ) | SLOPE µA/cm² × mM |
|---|---|
| 0 | 3.1 |
| 3 | 3.2 |
| 7 | 3.1 |
| 12 | 3.5 |
| 15 | 3.5 |

From the above results, it can be said that the thus prepared biosensors of the present invention show good linearity with an expanded concentration range of an analyte for a long period of time stably.

## Claims

1. An enzyme electrode which comprises
(a) an electroconductive support member (ESM) comprising a porous electroconductive layer,
(b) an enzyme adsorbed or immobilized onto the surface of said porous layer in an catalytically effective amount, and
(c) a protecting layer to prevent leaching of said enzyme from the porous layer.

2. The enzyme electrode according to claim 1, wherein said porous electroconductive layer is formed of carbon particles in intimate surface contact with finely divided particles of a platinum group metal and bonded together by a resin, said layer constituting a substantially heterogeneous porous substrate consisting essentially of resin-bonded metalised carbon particles with said metal particles distributed substantially uniformly therethroughout.

3. The enzyme electrode according to claim 2, wherein said platinum group metal is present in the form of particles more finely divided than said carbon particles.

4. The enzyme electrode according to claim 2, wherein said platinum group metal is present in the form of particles thereof adsorbed onto the surface of individual carbon particles, and said metal particles have colloidal sizes in the range of about 15 to 25 Angstroms.

5. The enzyme electrode according to claim 2, wherein said carbon particles have an average particle size of about 1 to about 10 Angströms.

6. The enzyme electrode according to claim 2, wherein said platinum group metal is present in an amount of from 1 to 100 % by weight based on the weight of said carbon particles.

7. The enzyme electrode according to claim 2, wherein said platinum group metal is present in an amount of from 5 to 80 % by weight based on the weight of said carbon particles.

8. The enzyme electrode according to claim 2, wherein said platinum group metal is platinum.

9. The enzyme electrode according to claim 2, wherein said platinum group metal is palladium.

10. The enzyme electrode according to claim 2, wherein said resin is selected from the group consisting of a fluorocarbon resin, a polyester resin, a cellulose, a polyamide resin and a poly(vinyl acetate) resin.

11. The enzyme electrode according to claim 1, wherein said porous electroconductive layer comprises an electroconductive polymer film and a layer comprising particles of a platinum group metal.

12. The enzyme electrode according to claim 11, wherein said electroconductive film is made of a polypyrrole.

13. The enzyme electrode according to claim 11, wherein the layer comprising particles of a platinum group metal is formed by electrochemical metallisation.

14. The enzyme electrode according to claim 11, wherein the layer comprising particles of a platinum group metal particles is formed by heat-pressing.

15. The enzyme electrode according to claim 11, wherein the layer comprising particles of a platinum group metal particles is formed by casting with a polymer binder.

16. The enzyme electrode according to claim 1, wherein said enzyme is immobilized or adsorbed in an amount of 10 to 3000 µg/cm² and preferably 30 to 1500 µg/cm².

17. The enzyme electrode according to claim 1, wherein said enzyme is an oxidoreductase.

18. The enzyme electrode according to claim 17, wherein said enzyme is selected from the group consisting of glucose oxidase, lactate oxidase, cholesterol oxidase, choline oxidase, glutamate oxidase, pyruvate oxidase, lactose oxidase, oxalate oxidase and sarcosine oxidase.

19. The enzyme electrode according to claim 17, wherein said oxidoreductase is accompanied by at least one enzyme other than the oxidoreductase.

20. The enzyme electrode according to claim 17, wherein said oxidoreductase is sarcosine oxidase and said enzyme other than oxidoreductase is creatininase with creatinase.

21. The enzyme electrode according to claim 17, wherein said protecting layer comprises at least one selected from the group consisting of gelatin, polyvinyl alcohol, poly(ethylene oxide), polyvinyl pyrrolidone, polyacrylamide and poly(2-hydroxyethyl methacrylate).

22. The enzyme electrode according to claim 21, wherein said protecting layer is cross-linked by using glutaraldehyde.

23. The enzyme electrode according to claim 21, wherein said protecting layer is cross-linked by using a photo cross-linker.

24. The enzyme electrode according to claim 1, wherein said electrode further comprises as a protective cover layer over said porous electroconductive layer and protective layer, which is a microporous membrane permeable to the detecting substance.

25. The enzyme electrode according to claim 24, wherein said membrane allows the permeation of said substance under diffusion-controlled conditions.

26. A process for indicating amperometrically the catalytic activity of an enzyme contained in the active coating of an enzyme electrode in the presence of a liquid containing a substance acted upon by said enzyme and of an electric potential on the electrode, the improvement which comprises an electrode having (a) an electroconductive support member comprising a porous electroconductive layer, (b) said enzyme adsorbed or immobilized onto the surface of said porous layer in an catalytically effective amount, and (c) a stabilizing layer to prevent leaching out of said enzyme from the porous layer.

27. An electroconductive polymer film which contains at least on one surface a layer of finely divided particles of a platinum group metal.

28. A polymer film according to claim 27, wherein the size of the particles is from 1 to 10 nm.

29. A polymer film according to claim 27, wherein the electroconductive polymer film is composed of a polyheteroaromatic polycation or a polyanaline polycation and non nucleophilic small anions selected from the group of halogenides, BF₄, PF₆, AsF₆, SbF₆, or polymeric polyanions of polymers with -SO₃⁻ or -OSO₃⁻groups.
